# EUROPEAN PATENT SPECIFICATION

(11) **EP 1 855 295 B1**
(45) Date of publication and mention of the grant of the patent: **28.03.2012**
(21) Application number: 06009426.5
(22) Date of filing: 08.05.2006
(51) Int. Cl.: H01F 1/44, A61K 41/00, A61N 1/40

(54) **Lipiodol-magnetic component and a process of preparation thereof**
Lipiodol-Magnetkomponente und zugehöriges Herstellungsverfahren
Lipiodol-composant magnétique et procédé de fabrication associé

(43) Date of publication of application: 14.11.2007
(73) Proprietor: Institute of Nuclear Energy Research Atomic Energy Council, Executive Yuan, Taoyuan County 32546, Taiwan (CN)
(72) Inventor: Chung, Jen-Chieh, Taoyuan County 325 (TW); Chen, Min-Nan, Wenshan District Taipei City 116 (TW); Huang, Ching-Tsuen, Taoyuan County 325 (TW)
(74) Representative: Negendanck, Matthias

(56) References cited:
- EP-A1- 1 604 673
- EP-A2- 0 913 167
- WO-A-00/52714
- US-A1- 2004 136 905
- MOROZ PAUL ET AL: "Histologic analysis of liver tissue following hepatic arterial infusion of ferromagnetic particles in a rabbit tumour model." BIOMETALS, vol. 16, no. 3, September 2003 (2003-09), pages 455-464, XP007901198 ISSN: 0966-0844
- MITSUMORI M ET AL: "Development of intra-arterial hyperthermia using a dextran-magnetite complex" International Journal of Hyperthermia UK, vol. 10, no. 6, 1994, pages 785-793, XP009073315 ISSN: 0265-6736
- DATABASE WPI Week 199407 Derwent Publications Ltd., London, GB; AN 1994-053914 XP002402500 -& JP 06 009411 A (MEITO SANGYO KK) 18 January 1994 (1994-01-18)

## Description

### Field of the Invention

The present invention relates Lipiodol-magnetic component, and a process for preparation thereof, wherein the Lipiodol-magnetic component includes the magnetic component including a magnetic component, γ-Fe₂O₃ or Fe₃O₄; and the Lipiodol. With the effect of external magnetic field, the Lipiodol-magnetic component can stay at a specific area in an organism, and then generated heat by high frequency waves, to achieve a high temperature treatment or diagnosis.

### Background of the Invention

Magnetic materials have been commonly and widely used in or applied to magnetic tapes and disks, recorders, magnetic switches, and seals, etc. Recent years, applying magnetic materials to new fields has been developing, the new fields including the medicine preparation, the purification of protein and DNA in biomedical field, and the management and treatment of environmental waste. For example, by using magnetic materials to result magnetically responsive spheres disclosed by US 4,687,748, the application of magnetic materials in cell separation, affinity purification or immunochemical assays is possible. The separation of a mixture to have the desired component or product is, therefore able to be reached by using a properly magnetic material, together with its related technical skills.

The magnetic materials, as mentioned above, are also so-called "ferrofluids" when they are dispensed in a liquid. In view of a target subject treated by such a magnetically separation technique, techniques with under external magnetic field, have been developed as follows: (1) when the sample having magnetic characteristic, separating the magnetically desired component from the sample, and (2) when the sample not having magnetic characteristic, prior to the process of separation, performing the combination of the sample with a magnetic material. The magnetic material in (2) will be a key in the combination process.

The process for preparing the magnetic material varies with different field and needs, and includes techniques of (1) mechanical grinding, for example, US4,604,222 disclosing the method of ball milling and grinding for preparing the ferrofluid composition by mixing ferromagnetic particles, a cationic surfactant, and organic liquid carriers such as glycol or ester, and then grinding the obtained mixture, the ferrofluid composition obtained being used to improve electrical conductivity in sealing computer disc drives and in sputtering apparatus in semiconductive industry; (2) oxidation, for example, US6,140,001 disclosing iron oxide magnetic particles prepared by oxidation of oxygen and ferrous (II) hydroxide, including by mixing a solution of a soluble phosphate compound, such as sodium orthophosphate, a solution of ferrous ion, with a solution of hydroxide of an alkali metal or of an alkaline earth metal; and (3) chemical co-precipitation, for example, US6,743,371 disclosing a magneto sensitive fluid composition exhibiting electrical switching as well as magnetorheological characteristics in the presence of external magnetic field, the composition prepared by mixing nickel-zinc ferrite or manganese-zinc ferrite and any conductive metallic or non metallic powder such as silver, graphite powder.

However, the disadvantages also exit in above processes, the timing for operation mechanical grinding taking around 2-6 weeks, chemical co-precipitation creating chemical waste, comprising un-reacted metal salt solutions and uncoated particles in aqueous and nonaqueous media needed to be disposed of in proper compliance with environmental regulations. The waste removal adds to the cost of manufacturing the ferrofluids.

Regarding the process for preparing magnetic materials, specifically, a ferrofluid, surface treatment of magnetic particles is necessary, so as to effectively avoid aggregation of particles due to attraction of magnetic characteristics thereof, and improve the dispersion in the liquid carrier. The way of surface treatment, for example, is different from water- or oil-based ferrofluids.

In terms of preparation of oil-based ferrofluids, US5,124,060 disclosing a magnetic fluid prepared by adding the low boiling organic solvent and the dispersant having oleophilic groups to fine ferromagnetic particles to obtain an intermediate medium, separating the fine particles of poor dispersibility from the intermediate medium and, then adding the less volatile organic solvent and heating the resulting material to evaporate the low boiling organic solvent. The magnetic fluid composition can be used for sealing devices in computer hard disk driver or vacuum apparatus. Another example disclosed by US6,068,785, the oil-based ferrofluid is prepared by grinding a slurry formed of particles of a non-magnetic oxide of iron α-Fe₂O₃, an oil carrier liquid, such as Ampro Type II oil, and a surfactant, such as polyolefin anhydride. Again, their non-economical impact is the disadvantage.

Furthermore, the application of conventionally oil-based ferrofluid, as mentioned previously, is primarily limited to magnetic memory device, sealing devices in computer hard disk driver or vacuum apparatus, but not to organism. While, in addition to the disadvantages of the process for preparing ferrofluid, as mentioned previously, α-Fe₂O₃ used in the process, as mentioned above, for preparing ferrofluid, is not a magnetic conductive material and is hard to perform magnetic transfer. Likewise, the unsuitable for many applications of water-based ferrofluids is left the room to improve the preparation and application of ferrofluid.
Further uses of ferrous materials used in organisms are described in the following documents:
Moroz, P., et al. describe in "Histologic analysis of liver tissue following hepatic arterial infusion of ferromagnetic particles in a rabbit tumour model", Biometals, vol. 16, no. 3, September 2003 (2003-09), pages 455-464, XP002402428, ISSN: 0966-0844 the preparation of the magnetic iron oxide particles (γ-Fe₂O₃) being suspended in lipiodol by ultrasonication to be embolized in rabbit organisms for histological analysis.
EP 0 913 167 A2 to Grey B. N. and Johnes S. K. describes the improved targeted hysteresis hyperthermia as a method for treating tissue. The herein described invention relates to an improved means for treating a patient's tissue using targeted hysteresis therapie. Therefore, a magnetic material having a magnetic heating efficiency is introduced in the patient organism, that magnetic material comprising a ferromagnetic material.
Then, WO 00/52714 A to Gray, B. and Jones, S. K. refers to another heating of magnetic material by hysteresis effects, being based on a substituted magnetite crystalline lattice in which some of the iron atoms in that crystalline lattice have been substituted for one or more alternate metal atoms.
Further, US 2004/0136905 A1 discloses magnetically guided particles for radiative therapies, that method being focused on an increase of the deposition of the desired energy in the targeted site. Solving of this problem shall be obtained by using magnetic component particles being suspended in a carrier material.
Mitsumori, et al. have been working on the "Development of intra-arterial hyperthermia using a dextran-magnetic complex", see: International Journal of Hyperthermia, 1994, pages 785-793, XP 0090737315, ISSN:0265-6736. Mitsumori et al. have found out that a Dextran-magnetite complex (DM) provides a specific absorption rate in a magnetic field, which is much higher than the absorption rate of multidomain ferrite particles. That specific absorption rate is due to the different mechanism of heat-generation, based on the Dextran coating of that magnetite.
Further, in EP 1 604 673 A1 to Uyama, H. et al. an auxiliary agent to be used in cancer therapy by dielectric heating is disclosed, which auxiliary agent is based on the use of a conductive substance, which may comprise ferrite compounds emulsified in oil containing liquids.
The DATABASE WPI, week 199407, Durwen Publications Ltd. London, Great Britain, points out compounds comprising a complex aq. solution, which is composed of water-soluble organic polymers and magnetic metals or magnetic metal compounds. These compounds are described to be useful as vascular embolic thermotherapeutic agents.

Accordingly, there is a need for a ferrofluid suitable for being used in an organism, and a process thereof is economical and efficient.

### Summary of the Invention

Accordingly, the aspect of this invention provides a Lipiodol magnetic component. as defined in claim 1.

Further aspects of this invention provide a process for preparing a Lipiodol-magnetic component as defined in claim 5 and a pharmaceutical composition as defined in claim 14.

Although a particular embodiment of the invention has been described in detail for purposes of illustration, various modifications and enhancements may be made without departing from the scope of the invention. Accordingly, the invention is not to be limited except as by the appended claims.

### Brief Description of the Drawings

FIG.1 is a process for preparing a magnetic component including a γ-Fe₂O₃ material according to a preferred embodiment of the present invention; and
FIG2 is a process for preparing a magnetic component including a Fe₃O₄ material according to a preferred embodiment of the present invention.

### Detailed Description of the Invention

The present invention mainly is to provide a Lipiodol-magnetic component, and a process for preparation thereof, wherein the Lipiodol-magnetic component includes the magnetic component including a magnetic component, γ-Fe₂O₃ or Fe₃O₄; and the Lipiodol. With the effect of external magnetic field, the Lipiodol-magnetic component can stay at a specific area in an organism, and then generated heat by high frequency waves, to achieve a high temperature treatment or diagnosis.

Lipiodol is manufactured by the esterification of ethanol and a fatty acid from the extracted oil of chestnut tree, and is widespread used as an X-ray contrast agent. With the latest findings of longer retention time or poor metabolic ability by Lipiodol in the liver cells of organism, the process for preparing Y-90- and Re-188-Lipiodol were disclosed by TW334438 and TW319773 of this Assignee/Applicant herein taken as references in this application, wherein the isotope labeled Lipiodol showed its efficiency in the treatment in radiation diagnosis.

The advantage of combination of magnetic component with Lipiodol being expected, as well as the process thereof, is capable of being shown by this invention. In order to make magnetic component dispersed in Lipiodol, the nano-grade magnetic component firstly is prepared, having a particle size ranging from 20-150nm, preferably 60-100nm. Adding Lipiodol to the magnetic component, and mixing well to be Lipiodol-magnetic component, while not influencing the features of Lipiodol, for example, the isotope labeled Lipiodol. Further, with the guidance by the effect of external magnetic field, the Lipiodol-magnetic component can stay at a specific area, and then generated heat by high frequency waves, so as to achieve the elimination of the target cells with a high temperature, around ranging from 38 Celcius Degree to 50 Celcius Degree, preferably 42 Celcius Degree, which causes the effect of hyperthermia to the target cells. Therefore, a pharmaceutical composition comprising the Lipiodol-magnetic component as defined in claim1 and a pharmaceutical acceptable carrier, could be manufactured.

One experimental process for preparing Lipiodol-magnetic component, specifically, comprising preparing oleophilic and nano-grade magnetic component by adding ferrous chloride aqueous solution to the mixture of Lipiodol and acetone, and then dripping slowly ethylendiamine aqueous solution while being heated to 60 Celcius Degree to 90 Celcius Degree, preferably 80 Celcius Degree for 3 hours, further dripping sodium nitrite aqueous solution and stirring constantly, the Lipiodol-magnetic component having a dark brown γ-Fe₂O₃ material then being obtained.

Another experimental process for preparing Lipiodol-magnetic component specifically, comprising preparing oleophilic and nano-grade magnetic component by adding ferrous sulphate aqueous solution to the solution of a metal complex agent, such as Diethylenetriaminepentaacetic acid (DTPA), and then dripping slowly the alkaline aqueous solution while being heated at a proper temperature and a period of time, the Lipiodol-magnetic component having a Fe₃O₄ material can be obtained.

The feature of the process is capable to conduct the process at low or general working-temperature, but no need through sintering, and by surface treating the ferrous material to combine oleophilic functional groups of a solution needed in the process. Furthermore, the final product of this invention of Lipiodol-magnetic component is easily prepared by aseptic filling the Lipiodol to the obtained magnetic component according this invention, by the ratio around 10% to 40% g/ml (magnetic component/Lipiodol, by weight/volume), preferably 30% g/ml (magnetic component/Lipiodol, by weight/volume), which is conveniently to be used in clinical application. Preferably, the weight to volume ratio is expressed as "g/ml".

More, Lipiodol of Lipiodol-magnetic component of this invention, before being mixed with magnetic component, could be labeled, as mentioned before, by isotopes, such as Y-90 and Re-188, so as to express the characteristics of tracing and radiation and magnetic property in an organism.

The above description, however, is only the preferable embodiment of the invention and cannot be used as a limitation for the scope of implementation of the invention. Any variation and modification made from the scopes claimed from the invention all should be included within the scope of the present invention, and hope your esteemed reviewing committee member examine this application favorably and permit it as a patent wishfully.

Example 1 Preparation of Lipiodol-magnetic component (γ-Fe₂O₃)

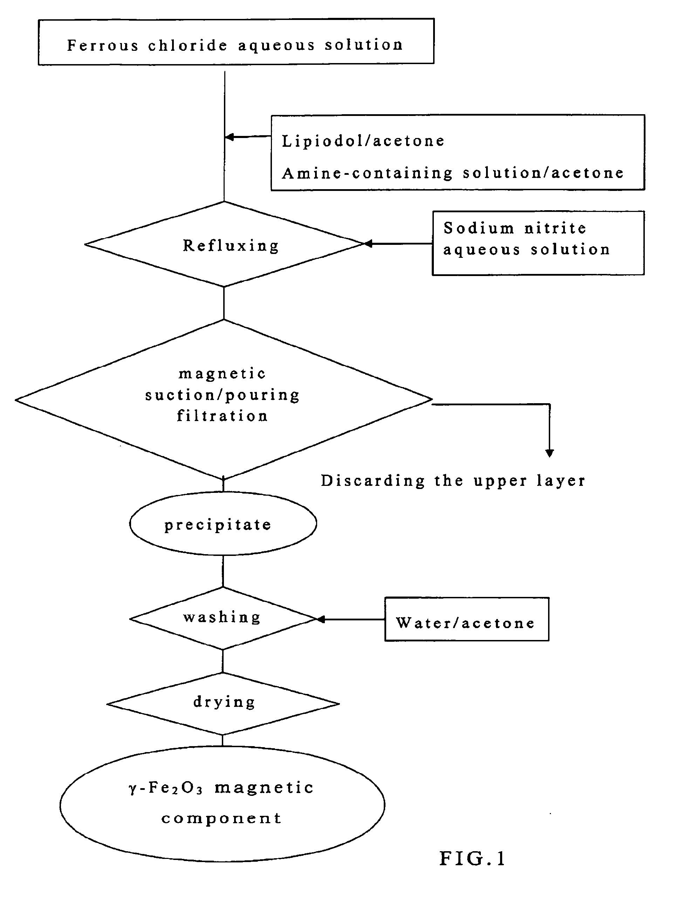

FIG 1 is a process for preparing a magnetic component including a γ-Fe₂O₃ material according to a preferred embodiment of the present invention. Mixing and stirring 0.5-1.5ml, preferably 1ml Lipiodol, with 15∼25ml, preferably 20ml acetone, and the solution of 4~8g, preferably 6g ferrous chloride (containing 2~6 hydrate, preferably 4 hydrate) (Merck, Germany) in 5~15ml, preferably 10ml water; during stirring, adding the mixture of 5~15ml, preferably 10ml of an amine-containing solution, such as Ethylendiamine solution, and 30~50ml, preferably 40ml acetone; refluxing at 60 to 90 Celcius Degree, preferably 80 Celcius Degree. Then, dripping slowly the solution of 5~15mg, preferably 10mg sodium nitrite in 10∼30ml, preferably 20ml water, and refluxing at 80 Celcius Degree at least one hour. Obtaining the precipitate by a proper way, such as magnetic suction/pouring filtration; washing the precipitate in order by 2~20ml, preferably 10ml of acetone, and reagent-grade water more than twice, then drying the washed precipitate. Aseptic filling the Lipiodol solution (Guerbet, France) to magnetic component, by the ratio of magnetic component to Lipiodol, around 10% to 40% (by weight to volume), preferably 30% (by weight to volume), to form pre-Lipiodol magnetic component. Finally, dispersing or vibrating the pre-Lipiodol magnetic component by ultrasound.

Lipiodol-magnetic component including a γ-Fe₂O₃ material according to a preferred embodiment of the present invention therefore is obtained. The particle size of the magnetic component, the γ-Fe₂O₃ material ranges from 20 to 150nm, preferably 60 to 100nm.

Ethylendiamine, mentioned above, could be replaced by hexamethylenetetramine.

Example 2 Preparation of Lipiodol-magnetic component (Fe₃O₄)

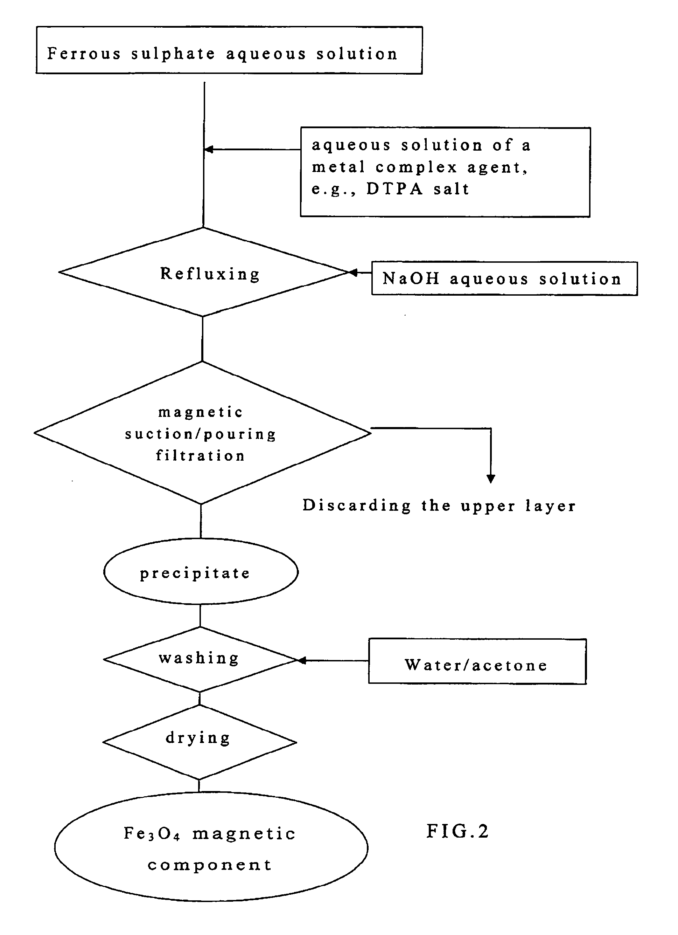

FIG.2 is a process for preparing a magnetic component including a Fe₃O₄ material according to a preferred embodiment of the present invention. Mixing and stirring the solution of 5∼9g, preferably 7.8g a metal complex agent, such as the salt of Diethylenetriaminepentaacetic acid (DTPA) in 5~15ml, preferably 10ml water, with the solution of 3~7g, preferably 5.5g ferrous sulphate (containing 5∼9hydrate, preferably 7 hydrate) (Merck, Germany) in 30~50ml, preferably 40ml water; refluxing at 80 to 120 Celcius Degree, preferably 110 Celcius Degree. During stirring, further dripping slowly the solution of 1~5g, preferably 3g alkaline material, such as sodium hydroxide in 5~20ml, preferably 10ml water, and refluxing at 110 Celcius Degree at least two hours. Cooling the refluxed mixture to ambient temperature; obtaining the precipitate by a proper way, such as magnetic suction/pouring filtration; washing the precipitate in order by 2~20ml, preferably 10ml of water and acetone more than twice; then drying the washed precipitate. Aseptic filling the Lipiodol solution (Guerbet, France) to the magnetic component, by the ratio of magnetic component to Lipiodol, around 10% to 40% g/ml (by weight to volume), preferably 30% g/ml (by weight to volume), to form pre-Lipiodol magnetic component. Finally, dispersing or vibrating the pre-Lipiodol magnetic component by ultrasound.

Lipiodol-magnetic component including a Fe₃O₄ material according to a preferred embodiment of the present invention therefore is obtained. The particle size of the magnetic component, the Fe₃O₄ material ranges from 20 to 150nm, preferably 60 to 100nm.

Diethylenetriaminepentaacetic acid, mentioned above, could be replaced by an acid selected from the group consisting of 1,2-diamino-cyclohexyl tetraacetic acid, nitril-triacetic acid, ethylenediaminetetraacetic acid, salicylic acid, lactic acid, gluconic acid, maleic acid, undecenoic acid, oleic acid, tartaric acid, lauric acid, and caproic acid. The alkaline material, mentioned above, could be replaced by either ammonia or pyridine.

## Claims

1. A Lipiodol-magnetic component, comprising:
(a) magnetic component, said magnetic component including a magnetic component having a particle size ranging from 20 to 100 nm and being selected from the group consisting of a γ-Fe₂O₃ material and a Fe₃O₄ material and
(b) Lipiodol;
wherein said magnetic component is surface treated and thereby adapted to combine with oleophilicgroups, and
wherein the weight to volume ratio of said magnetic component to said Lipiodol ranges from 10 to 40% g/ml.

2. The Lipiodol-magnetic component according to claim 1, with said magnetic component having a particle size ranging from 60 to 100 nm.

3. The Lipiodol-magnetic component according to claim 1 or 2, wherein said Lipiodol is labeled an isotope selected from the group consisting of Y-90 and Re-188.

4. The Lipiodol-magnetic component according to one of claims 1 to 3, wherein the weight to volume ratio of said magnetic component to said Lipiodol is 30 % g/ml.

5. A process for preparing Lipiodol-magnetic component, comprising steps of:
(a) preparing a magnetic component including a magnetic component having a particle size ranging from 20 to 100 nm and being one of a γ-Fe₂O₃ material and a Fe₃O₄ material, subjecting said magnetic component to surface treatment and thereby providing adaptation to combining with oleophilic groups, and,
(b) adding Lipiodol to the magnetic component by the weight to volume ratio of the ferrofluid to the Lipiodol ranging from 10 to 40 % g/ml; and
(c) dispersing the magnetic component in the Lipiodol.

6. The process according to claim 5, the magnetic component prepared in said step(a) having a magnetic component having a particle size ranging from 60 to 100 nm.

7. The process according to claim 5 or 6, wherein the weight to volume ratio of said magnetic component to said Lipiodol is 30 % g/ml.

8. The process according to one of claims 5 to 7, the magnetic component being γ-Fe₂O₃ and the step (a) includes sub-steps of:
(a1) mixing 0.5~1.5ml Lipiodol, with 15~25ml acetone, and a solution of 4~8g ferrous chloride (containing 2~6hydrate) in 5~15ml water to form a pre-mixture;
(a2) adding 5~15ml of an amine-containing solution and 30~50ml acetone to the pre-mixture;
(a3) subsequently dripping slowly a solution of 5~15mg sodium nitrite in 10~30ml water to the pre-mixture so as to form a mixture;
(a4) refluxing the mixture at 60 to 90 Celcius Degree; and
(a5) drying a precipitate formed from the refluxed mixture.

9. The process according to claim 8, the amine-containing solution used in said step (a2) being selected from the group consisting of ethylendiamine solution and hexamethylenetetramine solution.

10. The process according to one of claims 5 to 7, wherein the magnetic component used in said step(a) being a Fe₃O₄ material and said step (a) includes sub-steps of:
(a1) mixing a solution of 5~9g a metal complex agent in 5~15ml water, with a solution of 3~7g ferrous sulphate (containing 5∼9 hydrate) in 30~50ml water to form a pre-mixture;
(a2) refluxing the pre-mixture at 80 to 120 Celcius Degree;
(a3) subsequently dripping slowly a solution of 1~5g an alkaline material in 5~20ml water to the pre-mixture so as to form a mixture; and
(a4) drying a precipitate from the mixture.

11. The process according to claim 10, wherein the metal complex agent used in said step(a1) is selected from the group consisting of water-soluble salts of diethylenetriaminepentaacetic acid, 1,2-diamino-cyclohexyl tetraacetic acid, nitrile triacetic acid, ethylenediamine tetraacetic acid, salicylic acid, lactic acid, gluconic acid, maleic acid, undecenoic acid, oleic acid, tartaric acid, lauric acid, and caproic acid.

12. The process according to claim 10 or 11, wherein the alkaline material used in said step (a3) is selected from the group consisting of sodium hydroxide, ammonia, and pyridine.

13. The process according to claim 5, which further comprises labeling the Lipiodol by an isotope selected from the group consisting of Y-90 and Re-188 prior to said step (b).

14. A pharmaceutical composition comprising the Lipiodol-magnetic component as defined in claim 1 and a pharmaceutical acceptable carrier; wherein Lipiodol-magnetic component can generate heat by high frequency waves, so as to eliminate of the target cells with a high temperature, having a range from 38 Celcius Degree to 50 Celcius Degree.

## Patentansprüche

1. Lipiodol-magnetische Komponente, umfassend:
(a) Magnetische Komponente, wobei diese magnetische Komponente eine magnetische Komponente beinhaltet, welche eine Partikelgröße angeordnet zwischen 20 bis 100 nm aufweist und von der Gruppe bestehend aus einem γ-Fe₂O₃ Material und einem Fe₃O₄ Material ausgewählt ist und
(b) Lipiodol;
wobei die magnetische Komponente oberflächenbehandelt ist und **dadurch** angepasst ist mit oleophilen Gruppen zu kombinieren, und
wobei das Gewicht/Volumenverhältnis der magnetischen Komponente zu dem Lipiodol zwischen 10 zu 40 % g/ml angeordnet ist.

2. Lipiodol-magnetische Komponente nach Anspruch 1, mit der magnetischen Komponente aufweisend eine Partikelgröße, die zwischen 60 bis 100 nm angeordnet ist.

3. Lipiodol-magnetische Komponente nach Anspruch 1 oder 2, wobei das Lipiodol durch ein Isotop, ausgewählt von der Gruppe bestehend aus Y-90 und Re-188, markiert ist.

4. Lipiodol-magnetische Komponente nach einem der Ansprüche 1 bis 3, wobei das Gewicht/Volumenverhältnis der magnetischen Komponente zum Lipiodol 30 % g/ml ist.

5. Verfahren für das Zubereiten einer Lipiodol-magnetischen Komponente, umfassend die Schritte:
(a) Zubereiten einer magnetischen Komponente beinhaltend eine magnetische Komponente, welche eine Partikelgröße angeordnet zwischen 20 bis 100 nm aufweist und eine von einem γ-Fe₂O₃ Material und einem Fe₃O₄ Material ist, wobei die magnetische Komponente einer Oberflächenbehandlung unterzogen wird und **dadurch** eine Anpassung für das Kombinieren mit oleophilen Gruppen bereitstellt, und
(b) Zufügen von Lipiodol zu der magnetischen Komponente mit dem Gewicht/Volumenverhältnis von dem Ferrofluid zu dem Lipiodol angeordnet zwischen 10 zu 40 % g/ml; und
(c) Dispergieren der magnetischen Komponente in dem Lipiodol.

6. Verfahren nach Anspruch 5, wobei die magnetische Komponente vorbereitet in dem Schritt (a) eine magnetische Komponente beinhaltet, welche eine Partikelgröße angeordnet zwischen 60 bis 100 nm aufweist.

7. Verfahren nach Anspruch 5 oder 6, wobei das Gewicht/Volumenverhältnis der magnetischen Komponente zum Lipiodol 30% g/ml ist.

8. Verfahren nach Anspruch 5 bis 7, wobei die magnetische Komponente γ-Fe₂O₃ ist und der Schritt (a) die Unterschritte beinhaltet:
(a1) Mischen von 0,5 -1,5 ml Lipiodol mit 15 - 25 ml Aceton und einer Lösung von 4 - 8 g eisenhaltigen Chloriden (enthaltend 2 - 6 Hydrate) in 5 -15 ml Wasser, um eine Vormischung zu bilden;
(a2) Zufügen von 5 - 15 ml von einer Amin-enthaltenden Lösung und 30 bis 50 ml Aceton zu der Vormischung;
(a3) anschließend eine Lösung von 5 -15 mg Natriumnitrit in 10 - 30 ml Wasser zu der Vormischung langsam zu tropfen, um eine Mischung zu bilden;
(a4) Refluxieren der Mischung bei 60 bis 90 Grad Celsius; und
(a5) Trocknen einer Ablagerung gebildet von der refluxierten Mischung.

9. Verfahren nach Anspruch 8, wobei die Amin enthaltende Lösung aus Schritt (a2) von der Gruppe bestehend aus einer Ethylendiamin Lösung und einer Hexamethylentetetramine Lösung ausgewählt sind.

10. Verfahren nach Anspruch 5 bis 7, wobei die magnetische Komponente aus Schritt (a) ein Fe₃O₄ Material ist und der Schritt (a) die Unterschritte beinhaltet:
(a1) Mischen einer Lösung von 5 - 9 g eines Metallkomplexbildners in 5-15 ml Wasser, mit einer Lösung von 3 - 7 g eisenhaltigen Sulfaten (enthaltend 5 - 9 Hydrate) in 30 - 50 ml Wasser, um eine Vormischung zu bilden;
(a2) Refluxieren der Vormischung bei 80 bis 120 Grad Celsius;
(a3) anschließend eine Lösung von 1- 5 g alkalischen Materials in 5 - 20 ml Wasser zu der Vormischung langsam zu tropfen, um eine Mischung zu bilden;
(a4) Trocknen einer Ablagerung von der Mischung.

11. Verfahren nach Anspruch 10, wobei der Metallkomplexbildner aus Schritt (a1) von der Gruppe bestehend aus wasserlöslichen Salzen von Diethylentriaminpentaessigsäure, 1,2-Diamino-Cyclohexyl Tetraessigsäure, Nitrilotriessigsäure, Ethylendiamin, Tetraessigsäure, Salicylsäure, Milchsäure, Gluconsäure, Maleinsäure, Undecensäure, Ölsäure, Weinsäure, Laurinsäure und Capronsäure ausgewählt ist.

12. Verfahren nach Anspruch 10 oder 11, wobei das alkalische Material aus Schritt (a3) von der Gruppe bestehend aus Natriumhydroxid, Ammoniak und Pyridin ausgewählt ist.

13. Verfahren nach Anspruch 5, das ferner das Lipiodol beinhaltet, welches durch ein Isotop vor dem Schritt (b) markiert wurde, welches von der Gruppe bestehend aus Y-90 und Re-188 ausgewählt ist.

14. Pharmazeutische Zusammensetzung umfassend die Lipiodol-magnetische Komponente wie in Anspruch 1 beschrieben und einen pharmazeutischen tragfähigen Träger; wobei die Lipiodol-magnetische Komponente Hitze durch hohe Frequenzwellen generieren kann, um die Zielzellen mit einer hohen Temperatur zu eliminieren, aufweisend einen Bereich von 38 Grad Celsius zu 50 Grad Celsius.

## Revendications

1. Lipiodol-composant magnétique comprenant :
(a) un composant magnétique, ledit composant magnétique comprenant un composant magnétique possédant une taille de particule comprise dans la plage allant de 20 à 100 nm et étant choisi dans le groupe constitué par un matériau γ-Fe₂O₃ et un matériau Fe₃O₄ et
(b) du Lipiodol ;
dans lequel ledit composant magnétique est traité superficiellement et donc adapté pour être combiné à des groupes oléophiles, et
dans lequel le rapport poids sur volume dudit composant magnétique audit Lipiodol est compris dans la plage allant de 10 à 40 % g/ml.

2. Lipiodol-composant magnétique selon la revendication 1, ledit composant magnétique possédant une taille de particule comprise dans la plage allant de 60 à 100 nm.

3. Lipiodol-composant magnétique selon la revendication 1 ou 2, dans lequel ledit Lipiodol est marqué avec un isotope choisi dans le groupe constitué par Y-90 et Re-188.

4. Lipiodol-composant magnétique selon l'une quelconque des revendications 1 à 3, dans lequel le rapport poids sur volume dudit composant magnétique audit Lipiodol est de 30 % g/ml.

5. Procédé de préparation d'un Lipiodol-composant magnétique, comprenant les étapes consistant à :
(a) préparer un composant magnétique comprenant un composant magnétique possédant une taille de particule comprise dans la plage allant de 20 à 100 nm et étant choisi parmi un matériau γ-Fe₂O₃ et un matériau Fe₃O₄, soumettre ledit composant magnétique à un traitement superficiel et offrir ainsi une adaptation pour une combinaison à des groupes oléophiles, et
(b) ajouter le Lipiodol au composant magnétique avec le rapport poids sur volume du ferrofluide au Lipiodol compris dans la plage allant de 10 à 40 % g/ml ; et
(c) disperser le composant magnétique dans le Lipiodol.

6. Procédé selon la revendication 5, dans lequel le composant magnétique préparé dans ladite étape (a) comprend un composant magnétique possédant une taille de particule comprise dans la plage allant de 60 à 100 nm.

7. Procédé selon la revendication 5 ou 6, dans lequel le rapport poids sur volume dudit composant magnétique audit Lipiodol est de 30 % g/ml.

8. Procédé selon l'une quelconque des revendications 5 à 7, dans lequel le composant magnétique est le γ-Fe₂O₃ et l'étape (a) comprend les sous-étapes consistant à :
(a1) mélanger de 0,5 à environ 1,5 ml de Lipiodol avec 15 à environ 25 ml d'acétone, et une solution de 4 à environ 8 g de chlorure ferreux (contenant 2 à environ 6 hydrates) dans 5 à environ 15 ml d'eau pour former un pré-mélange ;
(a2) ajouter de 5 à environ 15 ml d'une solution contenant une amine et de 30 à environ 50 ml d'acétone au pré-mélange ;
(a3) ajouter ensuite lentement goutte à goutte une solution de 5 à environ 15 mg de nitrite de sodium dans 10 à environ 30 ml d'eau au pré-mélange afin de former un mélange ;
(a4) porter le mélange au reflux à 60 à 90 °C ; et
(a5) sécher un précipité formé à partir du mélange porté au reflux.

9. Procédé selon la revendication 8, dans lequel la solution contenant une amine utilisée dans ladite étape (a2) est choisie dans le groupe constitué par une solution d'éthylènediamine et une solution d'hexaméthylènetétramine.

10. Procédé selon l'une quelconque des revendications 5 à 7, dans lequel le composant magnétique utilisé dans l'étape (a) est un matériau Fe₃O₄ et ladite étape (a) comprend les sous-étapes consistant à :
(a1) mélanger une solution de 5 à environ 9 g d'un agent complexant de métal dans 5 à environ 15 ml d'eau, avec une solution de 3 à environ 7 g de sulfate ferreux (contenant 5 à environ 9 hydrates) dans 30 à environ 50 ml d'eau pour former un pré-mélange ;
(a2) porter le pré-mélange au reflux à 80 à 120 °C ;
(a3) ajouter ensuite lentement goutte à goutte une solution de 1 à environ 5 g d'un matériau alcalin dans 5 à environ 20 ml d'eau au pré-mélange afin de former un mélange ; et
(a4) sécher un précipité formé à partir du mélange.

11. Procédé selon la revendication 10, dans lequel l'agent complexant de métal utilisé dans ladite étape (a1) est choisi dans le groupe constitué par les sels solubles dans l'eau d'acide diéthylènetriaminepentaacétique, d'acide 1,2-diaminocyclohexyltétraacétique, d'acide nitriletriacétique, d'acide éthylènediaminetétraacétique, d'acide salicylique, d'acide lactique, d'acide gluconique, d'acide maléique, d'acide undécénoïque, d'acide oléique, d'acide tartrique, d'acide laurique, et d'acide caproïque.

12. Procédé selon la revendication 10 ou 11, dans lequel le matériau alcalin utilisé dans ladite étape (a3) est choisi dans le groupe constitué par l'hydroxyde de sodium, l'ammoniac et la pyridine.

13. Procédé selon la revendication 5, qui comprend en outre le marquage du Lipiodol par un isotope choisi dans le groupe constitué par Y-90 et Re-188 avant ladite étape (b).

14. Composition pharmaceutique comprenant le Lipiodol-composant magnétique tel que défini selon la revendication 1 et un support pharmaceutiquement acceptable, dans laquelle le Lipiodol-composant magnétique peut produire de la chaleur par des ondes de fréquence élevée afin d'éliminer les cellules cibles avec une température élevée comprise dans la plage allant de 38 °C à 50 °C.
